# EUROPÄISCHE PATENTSCHRIFT

(11) **EP 1 049 452 B1**
(45) Veröffentlichungstag und Bekanntmachung des Hinweises auf die Patenterteilung: **26.03.2003**
(21) Anmeldenummer: 99904746.7
(22) Anmeldetag: 07.01.1999
(51) Int. Cl.: A61K 7/48, A61K 7/42

(54) **LIPIDREDUZIERTE, FLIESSFÄHIGE KOSMETISCHE ODER DERMATOLOGISCHE ZUBEREITUNGEN**
REDUCED LIPID FLOWABLE COSMETIC OR DERMATOLOGICAL PREPARATIONS
PREPARATIONS COSMETIQUES OU DERMATOLOGIQUES COULANTES A TENEUR LIPIDIQUE REDUITE

(30) Priorität: 22.01.1998 DE 19802206
(43) Veröffentlichungstag der Anmeldung: 08.11.2000
(73) Patentinhaber: Beiersdorf Aktiengesellschaft, 20245 Hamburg (DE)
(72) Erfinder: BLECKMANN, Andreas, D-22926 Ahrensburg (DE); VON DER FECHT, Stephanie, D-22869 Schenefeld (DE); HAMER, Gunhild, D-20251 Hamburg (DE); SCHNEIDER, Günther, D-22607 Hamburg (DE)
(86) Internationale Anmeldenummer: EP9900041
(87) Internationale Veröffentlichungsnummer: WO99037282

(56) Entgegenhaltungen:
- DE-A- 2 511 600
- DE-A- 2 734 059
- DE-A- 3 820 693
- FR-A- 1 437 366
- G.PROSPERIO ET AL: "Neuere "essbare" O/W-Emulgator-Mischungen" RIECHSTOFFE AROMEN KOSMETICA (RAK)., Bd. 28, Nr. 1, 1978, Seiten 8-12, XP002112591 FACHVERLAG V. FRANKENSTEIN. ESCHERSHAUSEN., DE

## Beschreibung

Die vorliegende Erfindung betrifft kosmetische und dermatologische Zubereitungen, insbesondere solche vom Typ Öl-in-Wasser, Verfahren zu ihrer Herstellung sowie ihre Verwendung für kosmetische und medizinische Zwecke.

Die menschliche Haut übt als größtes Organ des Menschen zahlreiche lebenswichtige Funktionen aus. Mit durchschittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögem.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Natürlich ist dem Fachmann eine Vielzahl von Möglichkeiten bekannt, stabile O/W-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Der Stand der Technik kennt allerdings nur wenige Formulierungen, die so dünnflüssig sind, daß sie beispielsweise sprühbar wären.

FR-A-1 437 366 offenbart eine O/W Creme, deren öligen Phase 13% Glycerylstearatcitrat, 3% Cetylalkohol, und 8% pflanzliches Öl enthält, und deren wäßrigen Phase aus 70% destilliertes Wasser besteht, sowie eine O/W Creme, deren öligen Phase 25% Glycerylmyristatcitrat, 3% Cetylalkohol, und 10% pflanzliches Öl enthält, und die auch eine wäßrige Phase enthält.

Zudem haben dünnflüssige Zubereitungen des Standes der Technik häufig den Nachteil, daß sie instabil, auf einen engen Anwendungsbereich oder eine begrenzte Einsatzstoffauswahl begrenzt sind. Dünnflüssige Produkte, in denen beispielsweise stark polare Öle - wie die in handelsüblichen Produkten sonst häufig verwendeten Pflanzenöle - ausreichend stabilisiert sind, gibt es daher zur Zeit auf dem Markt nicht.

O/W-Emulsionen mit einer geringen Viskosität, die eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Eine Aufgabe der vorliegenden Erfindung war es, Zubereitungen zur Verfügung zustellen, welche eine sehr geringe Viskosität haben und nicht die Nachteile des Standes der Technik aufweisen.

Erstaunlicherweise werden diese Aufgaben gelöst durch dünnflüssige kosmetische oder dermatologische Zubereitungen, enthaltend
(I) einen oder mehrere partiell neutralisierte Ester von Monoglyceriden und/oder Diglyceriden gesättigter Fettsäuren mit Zitronensäure,
(II) einen oder mehrere Fettalkohole gewählt aus der Gruppe der verzweigten und unverzweigten Alkylalkohole mit 12 bis 40 Kohlenstoffatomen
(III) eine Wasserphase,
(IV) von 2 bis 7,5 Gew.% einer Lipidphase, bezogen auf das Gesamtgewicht der Zubereitungen,
wobei das Gewichtsverhältnis der Summe der Bestandteile aus (I) und (II) : (IV) aus dem Bereich von 20 : 1 bis 1 : 5 gewählt wird, und derer Viskosität bei 25°C kleiner ist als 2 500 mPa.s

Es war für den Fachmann daher nicht vorauszusehen gewesen, daß die erfindungsgemäßen Zubereitungen
- besser als feuchtigkeitsspendende Zubereitungen wirken,
- einfacher zu formulieren sein,
- besser die Hautglättung fördern,
- sich durch besser Pflegewirkung auszeichen,
- besser als Vehikel für kosmetische und medizinisch-dermatologische Wirkstoffe dienen
- bessere sensorische Eigenschaften, wie beispielsweise die Verteilbarkeit auf der Haut oder das Einzugsvermögen in die Haut, aufweisen würden
- höhere Stabilität gegenüber Zerfall in Öl- und Wasserphasen aufweisen und
- sich durch bessere Bioverträglichkeit auszeichnen würden
als die Zubereitungen des Standes der Technik.

Die erfindungsgemäßen Zubereitungen stellen daher eine Bereicherung des Standes der Technik in bezug auf fließfähige O/W-Emulsionen dar.

Erfindungsgemäß ist ferner die Verwendung von
(I) einem oder mehreren partiell neutralisierten Estern von Monoglyceriden und/oder Diglyceriden gesättigter Fettsäuren mit Zitronensäure,
(II) einem oder mehreren Fettalkoholen, gewählt aus der Gruppe der verzweigten und unverzweigten Alkylalkohole mit 12 bis 40 Kohlenstoffatomen,
(III) einer Wasserphase,
(IV) von 0 bis 10 Gew.% einer Lipidphase, bezogen auf das Gesamtgewicht der Zubereitungen,
wobei das Gewichtsverhältnis der Summe der Bestandteile aus (I) und (II) : (IV) aus dem Bereich von 20 : 1 bis 1 : 5 gewählt wird, und derer Viskosität bei 25°C kleiner its als 2 500 mPa·s zur Herstellung dünnflüssiger Zubereitungen, insbesondere von O/W-Emulsionen.

Der Lipidgehalt der erfindungsgemäß erhältlichen Zubereitungen kann vorteilhaft von 2 Gew.-% bis zu 7,5 Gew.-% variiert werden, wobei gleichermaßen günstige Ergebnisse erzielt werden. Im Falle der Lipidfreiheit liegt keine Emulsion sondern ein System vor, welches am treffendsten als Emulgatorgel bezeichnet werden sollte.

Bevorzugt enthalten erfindungsgemäße Zubereitungen bis zu 7,5 Gew.-% einer Lipidphase und stellen dann O/W-Emulsionen dar. Besonders vorteilhaft enthalten erfindungsgemäße Zubereitungen bis zu 6 Gew.-% einer Lipidphase. Besonders bevorzugt enthalten erfindungsgemäße Zubereitungen 2 bis 4 Gew.-% einer Lipidphase, insbesondere etwa 3 Gew.-%, jeweils bezogen auf das Gesamtgewicht der Zubereitungen.

Gemäß der hiermit vorgelegten Lehre sind O/W-Emulsionen erhältlich, deren Viskosität bei 25° C kleiner als 2500 mPa·s (= Millipascalsekunden), insbesondere kleiner als 2000 mPa·s ist (gemessen mit Viscotester VT-02, Haake).

Ein besonders vorteilhafter Citronensäureester ist das Glycerylstearatcitrat. Solche Citronensäureester sind beispielsweise erhältlich unter der Produktbezeichnung "IMWITOR® 370" der Gesellschaft Hüls AG.

Die Gesamtmenge an einem oder mehreren erfindungsgemäß verwendeten Glycerinestern von α-Hydroxycarbonsäuren und gesättigten Fettsäuren in den fertigen kosmetischen oder dermatologischen Zubereitungen wird vorteilhaft aus dem Bereich von 0,1 - 10,0 Gew.-% bevorzugt 0,5 - 6,0 Gew.-% gewählt, bezogen auf das Gesamtgewicht der Zubereitungen.

Bevorzugter, erfindungsgemäß verwendeter Fettalkohol ist der Cetyl-Stearylalkohol (ein Gemisch aus Hexadecanol-1 und Octadecanol-1 zu etwa gleichen Anteilen).

Die Gesamtmenge an einem oder mehreren erfindungsgemäß verwendeten Fettalkoholen in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Es ist erfindungsgemäß vorteilhaft, Gewichtsverhältnisse von einem oder mehreren partiell neutralisierten Estern von Monoglyceriden und/oder Diglyceriden gesättigter Fettsäuren mit Zitronensäure, einerseits und Fettalkoholen andererseits von 10 : 1 bis 1 : 5 zu wählen, bevorzugt von 6:1 bis 1 : 1, insbesondere bevorzugt von etwa 3 : 1.

Das Gewichtsverhältnis der Summe der Bestandteile aus einem oder mehreren partiell neutralisierten Estern von Monoglyceriden und/oder Diglyceriden gesättigter Fettsäuren mit Zitronensäure, und Fettalkoholen zu der Ölphase wird erfindungsgemäß aus dem Bereich von 20 : 1 bis 1 : 5 gewählt, vorteilhaft aus dem Bereich von 20 : 1 bis 1 : 2, insbesondere bevorzugt etwa 1 : 1.

Die Ölphase der erfindungsgemäßen O/W-Emulsionen wird vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl und dergleichen mehr.

Vorteilhaft werden die erfindungsgemäßen Öle ebenfalls gewählt aus der Gruppe Vaseline (Petrolatum), Paraffinöl und Polyolefine. Unter den Polyolefinen sind Polydecene die bevorzugten Substanzen.

Die Ölphase kann im Sinne der vorliegenden Erfindung ferner vorteilhaft gewählt werden aus der Gruppe der Ester aus gesättigten und/oder ungesättigten, ver-zweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, femer Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.Kombination.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Ψ-Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001- 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen O/W-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältem versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Derivate des 1,3,5-Triazins, vorzugsweise 2,4,6-Trianilino-(p-carbo-2'-ethyl-1'-hexyloxy) -1,3,5-triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtern zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

| **Beispiel 1 (O/W-Emulsion):** | |
|---|---|
| | Gew.-% |
| Glycerylstearatcitrat | 3,00 |
| Stearylalkohol | 1,00 |
| Octyldodecanol | 1,00 |
| Caprylic/Capric Triglyceride | 1,00 |
| Dicaprylylether | 1,00 |
| Carbomer | 0,15 |
| Glycerin | 3,00 |
| Parfüm, Konservierungsmittel, NaOH Farbstoffe, Antioxidantien etc. | q.s. |
| Wasser | ad 100,00 |
| pH-Wert eingestellt auf | 5,5 |

| **Beispiel 2 (O/W-Emulsion):** | |
|---|---|
| | Gew.-% |
| Glycerylstearatcitrat | 3,00 |
| Stearylalkohol | 1,00 |
| Octyldodecanol | 0,25 |
| Caprylic/Capric Triglyceride | 0,25 |
| Dicaprylylether | 0,25 |
| Carbomer | 0,15 |
| Glycerin | 3,00 |
| Parfüm, Konservierungsmittel, NaOH Farbstoffe, Antioxidantien etc. | q.s. |
| Wasser | ad 100,00 |
| pH-Wert eingestellt auf | 5,5 |

| **Beispiel 3 (O/W-Emulsion):** | |
|---|---|
| | Gew.-% |
| Glycerylstearatcitrat | 3,00 |
| Behenylalkohol | 1,00 |
| Dimethicon | 1,50 |
| Cycolmethicon | 1,50 |
| Carbomer | 0,15 |
| Glycerin | 6,00 |
| Parfüm, Konservierungsmittel, NaOH Farbstoffe, Antioxidantien etc. | q.s. |
| Wasser | ad 100,00 |
| pH-Wert eingestellt auf | 5,5 |

| **Beispiel 4 (O/W-Emulsion):** | |
|---|---|
| | Gew.-% |
| Glycerylstearatcitrat | 3,00 |
| Stearylalkohol | 1,00 |
| Octyldodecanol | 0,25 |
| Caprylic/Capric Triglyceride | 0,25 |
| Dicaprylylether | 0,25 |
| Dimethicon | 0,50 |
| Carbomer | 0,15 |
| Glycerin | 3,00 |
| Aluminium Starch Octenyl Succinate | 0,50 |
| Talkum | 0,50 |
| Bentonite | 0,50 |
| Parfüm, Konservierungsmittel, NaOH Farbstoffe, Antioxidantien etc. | q.s. |
| Wasser | ad 100,00 |
| pH-Wert eingestellt auf | 5,5 |

| **Beispiel 5 (O/W-Emulsion):** | |
|---|---|
| | Gew.-% |
| Glycerylstearatcitrat | 3,00 |
| Cetylalkohol | 1,00 |
| Squalan | 1,00 |
| Jojoba Öl | 1,00 |
| Paraffinum liquidum | 1,00 |
| Carbomer | 0,10 |
| Glycerin | 3,00 |
| Serin | 0,50 |
| Tocopherolacetat | 1,00 |
| Carbomer | 0,10 |
| Xanthangummi | 0,10 |
| Parfüm, Konservierungsmittel, NaOH Farbstoffe, Antioxidantien etc. | q.s. |
| Wasser | ad 100,00 |
| pH-Wert eingestellt auf | 6,0 |

| **Beispiel 6 (O/W-Emulsion):** | |
|---|---|
| | Gew.-% |
| Glycerylstearatcitrat | 3,00 |
| Cetylalkohol | 0,50 |
| Octyldodecanol | 0,40 |
| Caprylic/Capric Triglyceride | 0,40 |
| Dicaprylylether | 0,40 |
| Carbomer | 0,10 |
| Glycerin | 3,00 |
| Serin | 0,50% |
| Parfüm, Konservierungsmittel, NaOH Farbstoffe, Antioxidantien etc. | q.s. |
| Wasser | ad 100,00 |
| pH-Wert eingestellt auf | 5,5 |

| **Beispiel 7 (Emulsions-Make-up):** | |
|---|---|
| | Gew.-% |
| Glycerylstearatcitrat | 3,00 |
| Stearylalkohol | 1,00 |
| Dimethicon | 0,50 |
| Glycerin | 1,50 |
| 1,3 Butylenglycol | 1,50 |
| Magnesiumsilikat | 1,00 |
| Glimmer | 1,00 |
| Eisenoxide | 1,00 |
| Titandioxid | 2,50 |
| Talkum | 5,00 |
| Carbomer | 0,15 |
| Parfüm, Konservierungsmittel, NaOH, Farbstoffe, Antioxidantien etc. | q.s. |
| Wasser | ad 100,00 |
| pH-Wert eingestellt auf | 5,5 |

| **Beispiel 8 (O/W-Emulsion):** | |
|---|---|
| | Gew.-% |
| Glycerylstearatcitrat | 3,00 |
| Stearylalkohol | 1,00 |
| Octyldodecanol | 0,25 |
| Caprylic/Capric Triglyceride | 0,25 |
| Dicaprylylether | 0,25 |
| Octylmethoxycinnamat | 4,00 |
| Benzophenone-3 | 3,00 |
| Octylsalicylat | 3,00 |
| Carbomer | 0,15 |
| Glycerin | 3,00 |
| Parfüm, Konservierungsmittel, NaOH Farbstoffe, Antioxidantien etc. | q.s. |
| Wasser | ad 100,00 |
| pH-Wert eingestellt auf | 5,5 |

| **Beispiel 9 (O/W-Emulsion):** | |
|---|---|
| | Gew.-% |
| Glycerylstearatcitrat | 3,00 |
| Stearylalkohol | 1,00 |
| Octyldodecanol | 0,50 |
| Caprylic/Capric Triglyceride | 0,50 |
| Dicaprylylether | 0,50 |
| Distärkephosphat | 1,00 |
| Ethanol | 10,00 |
| Carbomer | 0,15 |
| Glycerin | 3,00 |
| Parfüm, Konservierungsmittel, NaOH Farbstoffe, Antioxidantien etc. | q.s. |
| Wasser | ad 100,00 |
| pH-Wert eingestellt auf | 5,5 |

| **Beispiel 10 (Emulgatorgel):** | |
|---|---|
| | Gew.-% |
| Glycerylstearatcitrat | 3,00 |
| Stearylalkohol | 1,00 |
| Ethanol | 2,00 |
| Aluminium Starch Octenyl Succinate | 0,25 |
| Talkum | 0,25 |
| Tapiokastärke | 0,25 |
| Carbomer | 0,15 |
| Glycerin | 3,00 |
| Parfüm, Konservierungsmittel, NaOH Farbstoffe, Antioxidantien etc. | q.s. |
| Wasser | ad 100,00 |
| pH-Wert eingestellt auf | 5,5 |

## Patentansprüche

1. Dünnflüssige kosmetische oder dermatologische Zubereitungen,
- deren Viskosität bei 25° C kleiner ist als 2500 mPa·s, enthaltend:
(l) einen oder mehrere partiell neutralisierte Ester von Monoglyceriden und/oder Diglyceriden gesättigter Fettsäuren mit Citronensäure,
(II) einen oder mehrere Fettalkohole gewählt aus der Gruppe der verzweigten und unverzweigten Alkylalkohole mit 12 bis 40 Kohlenstoffatomen
(III) eine Wasserphase,
(IV) von 2 bis 7,5 Gew.% einer Lipidphase, bezogen auf das Gesamtgewicht der Zubereitungen,
wobei das Gewichtsverhältnis der Summe der Bestandteile aus (I) und (II) : (IV) aus dem Bereich von 20 : 1 bis 1 : 5 gewählt wird.

2. Verwendung von
(I) einem oder mehreren partiell neutralisierten Estem von Monoglyceriden und/oder Diglyceriden gesättigter Fettsäuren mit Citronensäure,
(II) einem oder mehreren Fettalkoholen, gewählt aus der Gruppe der verzweigten und unverzweigten Alkylalkohole mit 12 bis 40 Kohlenstoffatomen,
(III) einer Wasserphase,
(IV) von 2 bis 7,5 Gew.% einer Lipidphase, bezogen auf das Gesamtgewicht der Zubereitungen,
wobei das Gewichtsverhältnis der Summe der Bestandteile aus (I) und (II) : (IV) aus dem Bereich von 20 : 1 bis 1 : 5 gewählt wird,
zur Herstellung dünnflüssiger Zubereitungen, insbesondere von O/W-Emulsionen,
- deren Viskosität bei 25° C kleiner ist als 2500 mPa·s.

3. Zubereitungen nach Anspruch 1 oder Verwendung nach Anspruch 2, **dadurch gekennzeichnet, dass** als partiell neutralisierter Ester von Monoglyceriden und/oder Diglyceriden gesättigter Fettsäuren mit Zitronensäure das Glycerylstearatcitrat gewählt wird.

4. Zubereitungen nach Anspruch 1 oder Verwendung nach Anspruch 2, **dadurch gekennzeichnet, dass** als Fettalkohol der Cetyl-Stearylalkohol gewählt wird.

5. Zubereitung nach Anspruch 1 oder Verwendung nach Anspruch 2, **dadurch gekennzeichnet, dass** die Gesamtmenge an einem oder mehreren partiell neutralisierten Estern von Monoglyceriden und/oder Diglyceriden gesättigter Fettsäuren mit Zitronensäure in den fertigen kosmetischen oder dermatologischen Zubereitungen gewählt wird aus dem Bereich von 0,1 - 10,0 Gew.-%, bevorzugt 0,5 - 6,0 Gew.-%, bezogen auf das Gesamtgewicht der Zubereitungen.

6. Zubereitungen nach Anspruch 1 oder Verwendung nach Anspruch 2, **dadurch gekennzeichnet, dass** die Gesamtmenge an einem oder mehreren erfindungsgemäß verwendeten Fettalkoholen in den fertigen kosmetischen oder dermatologischen Zubereitungen gewählt wird aus dem Bereich von 0,1 - 10,0 Gew.-%, bevorzugt 0,5 - 6,0 Gew.-%, bezogen auf das Gesamtgewicht der Zubereitungen.

7. Zubereitungen nach Anspruch 1 oder Verwendung nach Anspruch 2, **dadurch gekennzeichnet, dass** Gewichtsverhältnisse von einem oder mehreren partiell neutralisierten Estern von Monoglyceriden und/oder Diglyceriden gesättigter Fettsäuren mit Citronensäure einerseits und Fettalkoholen andererseits gewählt werden aus dem Bereich von 10 : 1 bis 1 : 10, bevorzugt von 2 : 1 bis 1 : 2, insbesondere bevorzugt von etwa 1 : 1.

8. Zubereitungen nach Anspruch 1 oder Verwendung nach Anspruch 2, **dadurch gekennzeichnet, dass** das Gewichtsverhältnis der Summe der Bestandteile aus partiell neutralisierten Estern von Monoglyceriden und/oder Diglyceriden gesättigter Fettsäuren mit Citronensäure und Fettalkoholen zu der Ölphase gewählt wird aus dem Bereich von 20 : 1 bis 1 : 5, vorteilhaft aus dem Bereich von 20 : 1 bis 1 : 2, insbesondere bevorzugt etwa 1 : 1.

## Claims

1. Low-viscosity cosmetic or dermatological preparations,
- the viscosity of which at 25°C is less than 2500 mPa.s, comprising:
(I) one or more partially neutralized esters of monoglycerides and/or diglycerides of saturated fatty acids with citric acid,
(II) one or more fatty alcohols chosen from the group of branched and unbranched alkyl alcohols having 12 to 40 carbon atoms,
(III) a water phase,
(IV) from 2 to 7.5% by weight of a lipid phase, based on the total weight of the preparations,
where the weight ratio of the sum of the constituents from (I) and (II) : (IV) is chosen from the range from 20 : 1 to 1 : 5.

2. Use of
(I) one or more partially neutralized esters of monoglycerides and/or diglycerides of saturated fatty acids with citric acid,
(II) one or more fatty alcohols chosen from the group of branched and unbranched alkyl alcohols having 12 to 40 carbon atoms,
(III) a water phase,
(IV) from 2 to 7.5% by weight of a lipid phase, based on the total weight of the preparations,
where the weight ratio of the sum of the constituents from (I) and (II) : (IV) is chosen from the range of from 20 : 1 to 1 : 5,
for the preparation of low-viscosity preparations, in particular of O/W emulsions,
- the viscosity of which at 25°C is less than 2500 mPa.s.

3. Preparations according to Claim 1 or use according to Claim 2, **characterized in that** the partially neutralized ester of monoglycerides and/or diglycerides of saturated fatty acids with citric acid chosen is glyceryl stearate citrate.

4. Preparations according to Claim 1 or use according to Claim 2, **characterized in that** the fatty alcohol chosen is cetylstearyl alcohol.

5. Preparation according to Claim 1 or use according to Claim 2, **characterized in that** the total amount of one or more partially neutralized esters of monoglycerides and/or diglycerides of saturated fatty acids with citric acid in the finished cosmetic or dermatological preparations is chosen from the range 0.1 - 10.0% by weight, preferably 0.5 - 6.0% by weight, based on the total weight of the preparations.

6. Preparations according to Claim 1 or use according to Claim 2, **characterized in that** the total amount of one or more fatty alcohols used according to the invention in the finished cosmetic or dermatological preparations is chosen from the range 0.1 - 10.0% by weight, preferably 0.5 - 6.0% by weight, based on the total weight of the preparations.

7. Preparations according to Claim 1 or use according to Claim 2, **characterized in that** weight ratios of one or more partially neutralized esters of monoglycerides and/or diglycerides of saturated fatty acids with citric acid on the one hand and fatty alcohols on the other hand are chosen from the range 10 : 1 to 1 : 10, preferably 2 : 1 to 1 : 2, particularly preferably of about 1 : 1.

8. Preparations according to Claim 1 or use according to Claim 2, **characterized in that** the weight ratio of the sum of constituents of partially neutralized esters of monoglycerides and/or diglycerides of saturated fatty acids with citric acid and fatty alcohols to the oil phase is chosen from the range 20 : 1 to 1 : 5, advantageously from the range 20 : 1 to 1 : 2, particularly preferably about 1 : 1.

## Revendications

1. Préparations fluides cosmétiques ou dermatologiques, dont la viscosité à 25°C est inférieure à 2500 mPa.s, contenant
(I) un ou plusieurs esters de monoglycérides et/ou de diglycérides d'acides gras saturés partiellement neutralisés avec de l'acide citrique,
(II) un ou plusieurs alcools gras choisis parmi le groupe des alcools alkyliques ramifiés et non ramifiés comprenant 12 à 40 atomes de carbone,
(III) une phase aqueuse,
(IV) 2 à 7,5% en poids d'une phase lipide par rapport au poids total des préparations, le rapport pondéral somme des constituants (I) et (II) à constituant (IV) étant choisi dans la plage de 20:1 à 1:5.

2. Utilisation
(I) d'un ou de plusieurs esters de monoglycérides et/ou de diglycérides d'acides gras saturés partiellement neutralisés avec de l'acide citrique,
(II) d'un ou de plusieurs alcools gras choisis parmi le groupe des alcools alkyliques ramifiés et non ramifiés comprenant 12 à 40 atomes de carbone,
(III) d'une phase aqueuse,
(IV) de 2 à 7,5% en poids d'une phase lipide, par rapport au poids total des préparations,
le rapport pondéral somme des constituants (I) et (II) à constituant (IV) étant choisi dans la plage de 20:1 à 1:5
pour la préparation de préparations fluides, en particulier d'émulsions H/E, dont la viscosité à 25°C est inférieure à 2500 mPa.s.

3. Préparations selon la revendication 1 ou utilisation selon la revendication 2, **caractérisées en ce qu'**on choisit le stéarate-citrate de glycéryle comme ester de monoglycérides et/ou de diglycérides d'acides gras partiellement neutralisés avec de l'acide citrique.

4. Préparations selon la revendication 1 ou utilisation selon la revendication 2, **caractérisées en ce qu'**on choisit l'alcool cétylique-stéarylique comme alcool gras.

5. Préparation selon la revendication 1 ou utilisation selon la revendication 2, **caractérisée en ce que** la quantité totale d'un ou de plusieurs esters de monoglycérides et/ou de diglycérides d'acides gras partiellement neutralisés avec de l'acide citrique dans les préparations cosmétiques ou dermatologiques finies est choisie dans la plage de 0,1 à 10,0% en poids, de préférence de 0,5 à 6,0% en poids, par rapport au poids total des préparations.

6. Préparations selon la revendication 1 ou utilisation selon la revendication 2, **caractérisées en ce que** la quantité totale d'un ou de plusieurs alcools gras utilisés selon l'invention dans les préparations cosmétiques ou dermatologiques finies est choisie dans la plage de 0,1 à 10,0% en poids, de préférence de 0,5 à 6,0% en poids, par rapport au poids total des préparations.

7. Préparations selon la revendication 1 ou utilisation selon la revendication 2, **caractérisées en ce que** les rapports pondéraux d'un ou de plusieurs esters de monoglycérides et/ou de diglycérides d'acides gras saturés partiellement neutralisés avec de l'acide citrique d'une part à alcools gras d'autre part sont choisis dans la plage de 10:1 à 1:10, de préférence de 2:1 à 1:2, de manière particulièrement préférée d'environ 1:1.

8. Préparations selon la revendication 1 ou utilisation selon la revendication 2, **caractérisées en ce que** les rapports pondéraux somme des constituants d'esters de monoglycérides et/ou de diglycérides d'acides gras saturés partiellement neutralisés avec de l'acide citrique et d'alcools gras à phase huileuse sont choisis dans la plage de 20:1 à 1:5, avantageusement dans la plage de 20:1 à 1:2, de manière particulièrement préférée d'environ 1:1.
